# FASCICULE DE BREVET EUROPEEN

(11) **EP 2 217 322 B1**
(45) Date de publication et mention de la délivrance du brevet: **21.08.2013**
(21) Numéro de dépôt: 08842542.6
(22) Date de dépôt: 20.10.2008
(51) Int. Cl.: A61N 1/05, A61N 1/36, A61B 5/04

(54) **DISPOSITIF DE STIMULATION D'UN TISSU VIVANT PAR MICROELECTRODES, SES MODULE AMOVIBLE ET UTILISATION**
VORRICHTUNG ZUR STIMULIERUNG VON LEBENDEM GEWEBE DURCH MIKROELEKTRODEN UND ENTFERNBARES MODUL UND SEINE VERWENDUNG
DEVICE FOR STIMULATING LIVING TISSUE BY MICROELECTRODES, AND REMOVABLE MODULE AND USE THEREOF

(30) Priorité: 22.10.2007 FR 0707369
(43) Date de publication de la demande: 18.08.2010
(73) Titulaire: Centre National de la Recherche Scientifique (CNRS), 75016 Paris (FR); Groupe Ecole Superieure D'ingenieurs En Electronique Et Electrotechnique, 93162 Noisy le Grand Cedex (FR); Université Bordeaux 1, 33405 Talence Cedex (FR)
(72) Inventeur: JOUCLA, Sébastien, F-33110 Le Bouscat (FR); YVERT, Blaise, F-33360 Carignan de Bordeaux (FR); ROUSSEAU, Lionel, F-94170 Le Perreux sur Marne (FR)
(74) Mandataire: Regimbeau
(86) Numéro de dépôt international: PCT/EP2008/064133
(87) Numéro de publication internationale: WO 2009/053333

(56) Documents cités:
- EP-A- 1 723 983
- WO-A-2005/087309
- US-A- 4 969 468
- US-A- 5 938 689
- US-A- 6 032 062
- US-A1- 2004 267 344
- US-A1- 2005 203 366
- US-A1- 2006 135 862
- US-A1- 2007 142 863
- US-B1- 7 006 859
- FROMHERZ PETER: "Sheet conductor model of brain slices for stimulation and recording with planar electronic contacts" EUROPEAN BIOPHYSICS JOURNAL, vol. 31, no. 3, juin 2002 (2002-06), pages 228-231, XP002474128 ISSN: 0175-7571 cité dans la demande
- HEUSCHKEL M O ET AL: "A three-dimensional multi-electrode array for multi-site stimulation and recording in acute brain slices" JOURNAL OF NEUROSCIENCE METHODS, ELSEVIER SCIENCE PUBLISHER B.V., AMSTERDAM, NL, vol. 114, 1 janvier 2002 (2002-01-01), pages 135-148, XP002425883 ISSN: 0165-0270 cité dans la demande
- MCINTYRE C C ET AL: "Finite element analysis of the current-density and electric field generated by metal microelectrodes" ANNALS OF BIOMEDICAL ENGINEERING BIOMED. ENG. SOC USA, vol. 29, no. 3, mars 2001 (2001-03), pages 227-235, XP002474127 ISSN: 0090-6964

## Description

L'invention concerne un dispositif de stimulation d'un tissu vivant par une matrice de microélectrodes.

Les matrices de microélectrodes sont utilisées par exemple dans des applications neurophysiologiques et pharmacologiques.

Ces matrices sont utilisées pour enregistrer les activités électriques d'un tissu vivant (activités cellulaires ou multicellulaires). Elles sont également utilisées pour stimuler électriquement un tissu. Ceci s'applique pour tout type de tissu excitable, notamment tissu nerveux, tissu musculaire, par exemple cardiaque, ou cellules souches.

On cherche notamment à pouvoir stimuler un tissu nerveux de manière focale.

Une application à terme concerne les neuroprothèses, et vise à mettre au point des microstimulateurs implantés dans le corps (notamment humain, mais également chez l'animal) pour générer des stimulations électriques, afin de compenser des dysfonctionnements des réseaux neuronaux dans le cas d'une maladie ou d'une anomalie d'un organe. Il s'agit par exemple de stimulations profondes du cerveau dans le cas de maladies neurodégénératives (par exemple Parkinson, Alzheimer, démences), de la moelle épinière dans le cas de troubles moteurs ou de traitement de la douleur par exemple, des muscles, ou encore des structures sensorielles comme les nerfs périphériques, la rétine, la cochlée (oreille interne), ou autres relais sensoriels.

Pour pouvoir stimuler des neurones vivants, la matrice de microélectrodes doit donc être dimensionnée à l'échelle du tissu vivant à stimuler.

Les microélectrodes de stimulation ont habituellement un diamètre de l'ordre de quelques dizaines de micromètres ou moins et un espacement de quelques centaines de micromètres ou moins. Cependant, pour la stimulation plus macroscopique du système nerveux central ou périphérique, des électrodes plus grandes (de l'ordre du mm ou du cm) sont utilisées.

Les documents [1] à [6] mentionnés ci-dessous décrivent des matrices d'électrodes de stimulation, ainsi que des méthodes pour les utiliser. La matrice peut comporter plusieurs dizaines ou plusieurs centaines de microélectrodes de stimulation.

Le document [1] prévoit ainsi une matrice ayant 36 microélectrodes de stimulation et 4 électrodes de référence.

Le document [5] prévoit quant à lui que les microélectrodes soient réparties en deux groupes, l'un utilisé pour la stimulation et l'autre utilisé pour l'enregistrement, ce qui double le nombre de microélectrodes.

L'un des problèmes rencontrés avec les microélectrodes est d'obtenir une stimulation focale du tissu vivant. En effet, dans la matrice de microélectrodes de stimulation, si l'on envoie un signal de stimulation à l'une des microélectrodes, c'est pour stimuler la zone du tissu nerveux située en vis-à-vis de cette microélectrode.

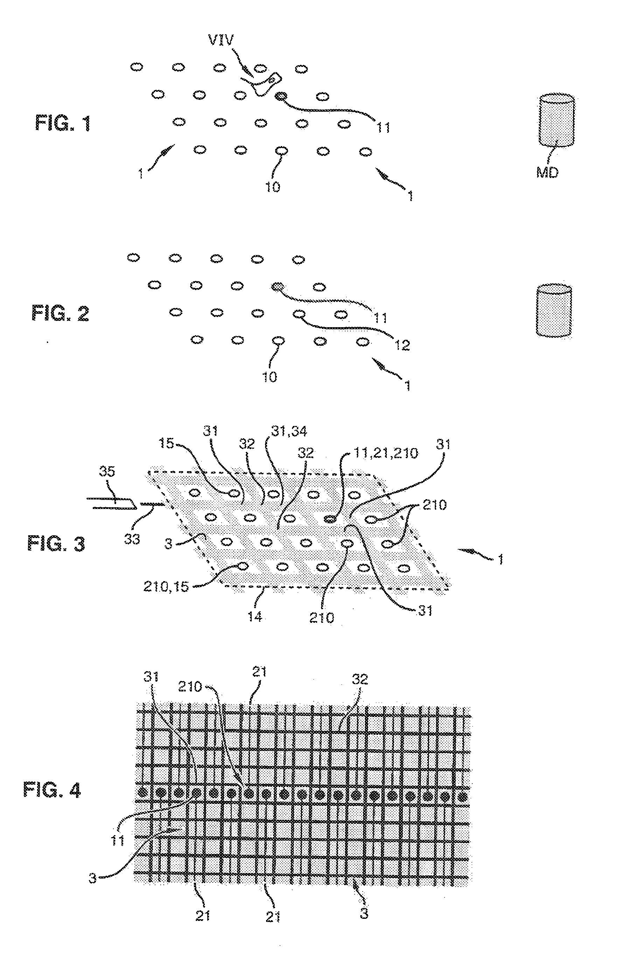

On connaît ainsi les stimulations monopolaires, dans lesquelles la stimulation d'une cellule vivante VIV s'effectue entre une microélectrode de stimulation 11 parmi les microélectrodes 10 de stimulation de la matrice 1 et une masse distante MD de la matrice 1 de microélectrodes de stimulation, ainsi que cela est représenté à la figure 1.

Dans le cas d'une stimulation bipolaire, ainsi que cela est représenté à la figure 2, la stimulation est effectuée entre deux microélectrodes de stimulation 11&12 de la matrice 1.

Ces deux types de stimulation ne sont toutefois pas satisfaisants, dans la mesure où, pour une stimulation monopolaire, une électrode loin d'un neurone peut quand même activer ce neurone avec le même courant qu'une électrode proche de ce neurone, et où, pour une stimulation bipolaire, il existe une zone aveugle où des neurones proches des électrodes stimulantes ne sont pas excités.

Le document [8] WO 2005/087309 décrit une disposition d'électrodes pour l'excitation de nerfs ou de muscles qui consiste à remplacer des électrodes de grandes tailles par un groupe d'électrodes de plus petites tailles occupant au total une dimension comparable à celle de la grande électrode. Ces groupes forment un unique site de stimulation. Chaque site de stimulation comporte trois ou cinq groupes constitués chacun d'éléments surfaciques conducteurs de l'électricité qui sont reliés entre eux par des pistes conductrices. Une matrice d'électrodes comporte sept sites de stimulation ayant chacun cinq groupes constitués chacun d'éléments surfaciques conducteurs de l'électricité qui sont reliés entre eux par des pistes conductrices. L'utilisation de groupes d'électrodes pour chaque site de stimulation permet d'obtenir une distribution de potentiel plus homogène dans la zone stimulée en regard du site de stimulation que celle obtenue avec une seule électrode de grande taille. Ceci entraine une homogénéisation des stimulations, ce qui joue au détriment de leur focalisation.

Le document [7] prévoit un procédé de stimulation préférentielle de somas neuraux, prévoyant de positionner au voisinage de la région du tissu neural une électrode de stimulation comprenant une première région intérieure conductrice en forme de disque, entourée par une deuxième région conductrice annulaire, les première et deuxième régions conductrices étant séparées par une région isolante. Le courant de stimulation est délivré entre le conducteur circulaire central et le conducteur annulaire, ce dernier fournissant une boucle de retour de courant. La dispersion latérale du courant dans le tissu neural est contenue dans une zone plus locale qu'avec une stimulation monopolaire, de sorte que le nombre de somas activés par l'électrode est limité, incluant uniquement ceux proches de l'électrode centrale.

Ce dispositif présente le double inconvénient de devoir doubler la connectique de l'électrode de stimulation, et de devoir délivrer des courants plus importants pour parvenir à stimuler les cellules locales.

L'invention vise à pallier ces inconvénients pour une matrice de microélectrodes de stimulation, disposées selon une configuration déterminée côté à côte et sélectionnables pour l'application d'un signal électrique de stimulation par l'une des microélectrodes.

En particulier, le dispositif de stimulation doit pouvoir être généralisé à un grand nombre de microélectrodes de stimulation dans la matrice, tout en étant simple à mettre en oeuvre.

A cet effet, l'invention a pour objet un dispositif de stimulation d'un tissu vivant, comprenant une matrice de microélectrodes, disposées selon une configuration déterminée côte à côte et sélectionnables pour l'application d'un signal électrique de stimulation par l'une des microélectrodes, les microélectrodes étant isolées l'une de l'autre et comportant chacune un conducteur d'envoi d'un signal de stimulation, ayant une section d'application locale au tissu vivant,
caractérisé en ce qu'il comporte, en plus des conducteurs des microélectrodes, au moins une surface conductrice supplémentaire d'application en tout ou partie contre le tissu vivant, qui est isolée des conducteurs des microélectrodes, et comporte une pluralité de zones conductrices situées respectivement au voisinage d'une pluralité déterminée de sections d'application locale de microélectrodes de la matrice,
des moyens de liaison étant prévus pour assurer une liaison électrique entre les zones conductrices de la surface supplémentaire,
la surface conductrice supplémentaire étant reliée en outre à au moins un accès destiné à être relié à un conducteur extérieur de retour au moins partiel du signal de stimulation et étant formée pour assurer une stimulation focale à partir d'au moins une de la pluralité déterminée de microélectrodes.

Suivant des modes de réalisation de l'invention :
- La surface conductrice supplémentaire est intégrée au même support que celui des microélectrodes.
- Ou la surface conductrice supplémentaire est intégrée sur un support différent de celui des microélectrodes.
- La surface conductrice supplémentaire est en forme de grille, dont les zones conductrices sont formées par des mailles passant autour de microélectrodes, les moyens de liaison étant formés sur la surface supplémentaire par les intersections des mailles entre elles.
- Lesdites mailles passant autour de microélectrodes entourent chacune une seule microélectrode.
- La grille est formée par des lignes rectilignes sécantes.
- Chacune des mailles de la grille entourant une microélectrode forme un pixel de stimulation restreint à l'espace délimité par cette maille.
- La surface conductrice supplémentaire passe entre des microélectrodes.
- La surface conductrice supplémentaire entoure des microélectrodes.
- La surface conductrice supplémentaire est continue avec des ouvertures de passage des sections d'application locale de microélectrodes.
- La surface conductrice supplémentaire comporte des branches électriquement en parallèle entre des microélectrodes et passant chacune au voisinage de plusieurs microélectrodes.
- Les moyens de liaison électrique entre les différentes zones conductrices sont situés au moins en partie dans la surface conductrice supplémentaire.
- Les moyens de liaison électrique sont situés au moins en partie à l'extérieur de la surface conductrice supplémentaire, à l'intérieur ou à l'extérieur d'un support de la surface conductrice supplémentaire ou dans un circuit électrique extérieur supplémentaire.
- La surface conductrice supplémentaire présente une conductivité électrique surfacique d'interface (conductivité d'interface entre l'électrode et le tissu) supérieure ou égale à 100 S/m² à une fréquence de 100 Hz à 1000 Hz.
- La surface conductrice supplémentaire présente une conductivité électrique surfacique d'interface (conductivité d'interface entre l'électrode et le tissu) supérieure ou égale à 1000 S/m² à une fréquence de 100 Hz à 1000 Hz, et de préférence supérieure ou égale à 40000 S/m² à une fréquence de 100 Hz à 1000 Hz.
- La matrice de microélectrodes possède un pas d'écartement entre microélectrodes, et lesdites zones conductrices de la surface supplémentaire passent à une distance de la pluralité des microélectrodes, inférieure ou égale à cinq fois le pas d'écartement maximum entre microélectrodes, et de préférence à une distance inférieure ou égale au pas d'écartement minimum entre microélectrodes.
- Lesdites zones conductrices de la surface supplémentaire passent à une distance de la pluralité des microélectrodes, inférieure ou égale à 500 µm.
- Le dispositif comporte une multiplicité d'accès électriques aux microélectrodes, qui sont associés respectivement à la multiplicité de microélectrodes de la matrice, l'accès de la surface conductrice (3) supplémentaire étant unique et distinct des accès électriques des microélectrodes.
- Le dispositif comporte une multiplicité d'accès électriques aux microélectrodes, qui sont associés respectivement à la multiplicité de microélectrodes de la matrice, l'accès de la surface conductrice supplémentaire étant multiple et distinct des accès électriques des microélectrodes .
- Le dispositif comporte une multiplicité d'accès électriques aux microélectrodes, qui sont associés respectivement à la multiplicité de microélectrodes de la matrice, l'accès de la surface conductrice supplémentaire étant unique et distinct des accès électriques des microélectrodes, ceci pour chaque surface conductrice supplémentaire dans le cas de leur pluralité.
- Les microélectrodes situées au bord de la matrice délimitent une région d'application contre le tissu vivant et le bord ou l'accès de la surface conductrice supplémentaire est situé à l'extérieur de la région d'application des microélectrodes.
- Le dispositif comporte en outre un premier système de génération de stimulus électriques ou courants de stimulation et de délivrance de ceux-ci au tissu via les microélectrodes, et également d'amplification et de multiplexage des signaux enregistrés avec les microélectrodes. Ce système est relié aux microélectrodes et à l'accès de la surface. Le dispositif comporte également un deuxième système d'acquisition et de contrôle muni d'une interface homme - machine pour la commande du premier système afin d'envoyer à au moins l'une présélectionnée des microélectrodes un signal de stimulation prédéterminé sur l'interface homme - machine, et de recueillir l'activité du tissu vivant en réponse ou non au signal de stimulation et la restituer sur l'interface homme - machine.
- Les microélectrodes situées au bord de la matrice délimitent une région d'application contre le tissu vivant et l'accès de la surface est situé à l'intérieur de la région d'application des microélectrodes.
- Le dispositif est mis en oeuvre au contact d'un tissu vivant, ou une partie d'un organe vivant, in vivo ou in vitro, une préparation cellulaire, un explant, un organisme vivant, un système de laboratoire, un organe vivant isolé, une partie d'organe vivant isolé, ou un implant pour un être vivant.

Un deuxième objet de l'invention est un ensemble amovible destiné à être monté dans le dispositif tel que décrit ci-dessus, caractérisé en ce qu'il comporte sur un même module amovible ou répartis sur plusieurs modules amovibles séparés la matrice de microélectrodes, ladite surface supplémentaire, et un circuit d'interface électrique d'entrée-sortie pour la connexion électrique des microélectrodes et de la surface supplémentaire avec l'extérieur, comportant une multiplicité de bornes d'accès électriques à respectivement la multiplicité de microélectrodes de la matrice et une borne d'accès de surface supplémentaire, distincte des bornes d'accès électriques des microélectrodes.

Un troisième objet de l'invention est une utilisation du dispositif tel que décrit ci-dessus pour l'enregistrement de signaux émis par un tissu vivant.

L'invention sera mieux comprise à la lecture de la description qui va suivre, donnée uniquement à titre d'exemple non limitatif en référence aux dessins annexés, sur lesquels :
- les figures 1 et 2 sont des vues schématiques en perspective d'exemples connus de matrice de microélectrodes,
- les figures 3, 5, 6, 7, 8, 9 sont des vues schématiques en perspective de premier, deuxième, troisième, quatrième, cinquième et sixième modes de réalisation d'une matrice de microélectrodes de stimulation suivant l'invention,
- la figure 4 est une vue d'un exemple de réalisation d'une matrice linéaire de microélectrodes de stimulation suivant l'invention,
- la figure 10 représente un système électronique de mesure et de stimulation pouvant être utilisé avec la matrice de microélectrodes suivant l'invention.
- la figure 11 est un diagramme montrant le potentiel électrique en fonction de la distance par rapport à une microélectrode stimulante pour différents types de stimulation,
- la figure 12 est un diagramme montrant le potentiel électrique en fonction de la distance par rapport à une microélectrode stimulante pour différentes types configuration, et
- la figure 13 est un diagramme montrant le potentiel électrique en fonction de la distance par rapport à une microélectrode stimulante pour différentes conductivité surfaciques.

La stimulation électrique extracellulaire d'un tissu vivant consiste à faire passer un courant électrique à travers une combinaison d'électrodes placées au contact du tissu. Certaines électrodes injectent un courant positif dans le tissu, alors que d'autres injectent simultanément un courant négatif dans le tissu, de telle sorte que la somme des amplitudes des courants positifs est égale à la somme des amplitudes des courants négatif. Afin d'injecter ces courants, des valeurs de potentiel sont appliquées aux électrodes. Ces valeurs sont déterminées par rapport à une électrode de masse qui, par définition, est au potentiel 0V.

La stimulation la plus simple est la stimulation monopolaire. Celle-ci consiste à injecter un courant (positif ou négatif) dans une seule électrode : un potentiel est appliqué à une seule électrode. Ainsi, le retour de courant s'effectue par la masse qui est au potentiel 0 V. Classiquement, la masse est située à une grande distance des électrodes de stimulation, en général de l'ordre du mm.

Afin d'obtenir des stimulations plus focales spatialement, des stimulations multipolaires sont classiquement utilisées : non plus une seule, mais plusieurs électrodes sont utilisées en combinaison sur lesquelles sont appliqués des potentiels différents (d'où le terme multipolaire). Par exemple, une stimulation bipolaire est utilisée en appliquant des valeurs de potentiel différentes sur 2 électrodes généralement situées côte à côte, de telle sorte qu'en général les courants traversant les deux électrodes sont d'amplitudes identiques et de signes opposés. De même, une stimulation tripolaire utilise 3 électrodes, en général de telle sorte que le courant injecté par une électrode centrale revient à parts égales par les deux autres l'entourant. Dans les configurations multipolaires, si les niveaux de potentiel appliqués aux différentes électrodes ne permettent pas aux courants positifs et négatifs de se compenser parfaitement, un courant résiduel reviendra par l'électrode de masse.

Lors d'une stimulation multipolaire, plusieurs électrodes portées à des potentiels différents sont utilisées en combinaison. Cependant, malgré la pluralité des électrodes mises en jeu, ce groupe d'électrodes constitue une unique unité de stimulation. L'utilisation de matrices d'électrodes permet de construire des matrices contenant plusieurs unités de stimulation. Le cas le plus simple est celui pour des stimulations monopolaires où chaque électrode de la matrice constitue (avec la masse) une unité. Il y a dans ce cas autant d'unités que d'électrodes. Afin de focaliser les stimulations, chaque unité de stimulation peut être constituée de plusieurs électrodes. Par exemple, il est possible de créer des unités bipolaires constituées de paires d'électrodes, mais cette approche a cependant l'inconvénient de devoir dédoubler le nombre de microélectrodes de la matrice pour disposer du même nombre d'unités de stimulation.

Un des intérêts de l'invention est de disposer d'un nombre d'unités de stimulation égal au nombre d'électrodes de la matrice, tout en obtenant un gain en focalité des stimulations. Un autre avantage est de ne pas perdre beaucoup en amplitude de stimulation.

Aux figures 3 à 10, chaque microélectrode 11 de la matrice 1 comporte un conducteur 21 d'application destiné à être appliqué contre un tissu vivant et est apte à servir de microélectrode de stimulation, pour lui envoyer un signal de stimulation. Dans ce conducteur 21 passe donc le courant de stimulation. Ce conducteur est isolé des conducteurs des autres électrodes, et est isolé en partie du tissu vivant. Chaque conducteur 21 de microélectrode comporte une section 210 d'application locale au tissu vivant, qui est sa partie tournée vers le tissu vivant et devant toucher celui-ci, par exemple formée par l'extrémité du conducteur 21 et par exemple circulaire. Chaque conducteur 21 de microélectrode comporte également un accès 13 extérieur généralement individuel, permettant la connexion d'un circuit électrique au support 40 de la matrice 1. Le pas d'écartement entre les microélectrodes 11 peut être fixé à une valeur déterminée pour toute la matrice ou avoir des valeurs différentes pour des régions différentes de la matrice. Les accès 13 sont en général isolés entre eux au niveau du support de la matrice.

Une ou plusieurs surfaces conductrices supplémentaires 3 sont disposées à proximité de certaines ou de toutes les microélectrodes 11. Chaque surface 3 et les microélectrodes 11 sont positionnées pour pouvoir être appliquées conjointement contre le tissu vivant, et la surface 3 sert à retourner le courant de stimulation envoyé par une microélectrode voisine en le focalisant à proximité de celle-ci. Les sections 210 et la surface 3 sont en général tournées vers un même côté vis à vis du tissu. Les accès 13 aux microélectrodes 11 de stimulation, les conducteurs 21 et les sections 210 sont électriquement isolés de la surface 3, par exemple en passant dans des couches différentes de celle(s) de la surface 3, au-dessus ou au-dessous de celle-ci. Les sections conductrices 210 et la surface conductrice 3 sont nues du côté d'application contre ou dans le tissu vivant.

La surface 3 comporte donc des zones conductrices 31 localisées au voisinage de certaines sections 210 d'application de microélectrodes, qui sont reliées entre elles pour être sensiblement équipotentielles.

La surface 3 sert de moyen de focalisation en commun pour plusieurs sections 210 d'application de microélectrodes 11 différentes.

Suivant l'invention, la grille ou surface supplémentaire 3 n'est pas au même potentiel que les microélectrodes stimulantes 11, ce qui n'est pas le cas pour le document [8] WO 2005/087 309, où le but est d'homogénéiser la stimulation et donc où le potentiel est le même sur toutes les microélectrodes du même site de stimulation.

Chaque microélectrode 11 forme en général un unique site de stimulation. Toutefois, plusieurs microélectrodes peuvent être sélectionnées simultanément pour la stimulation, ce qui correspond à l'utilisation simultanée de plusieurs sites de stimulation. Chaque section 210 d'application locale forme en général un unique site de stimulation. La surface supplémentaire 3 ou grille est mise par son accès 33, 35 à un potentiel électrique généralement différent du potentiel envoyé aux microélectrodes 11 par leur accès 13, par exemple pour une stimulation par la section 210 d'application de la microélectrode 11 ayant été sélectionnée. Le potentiel de la surface supplémentaire 3 est en général fixé à zéro Volt mais peut être fixé à une valeur différente de zéro Volts. La surface supplémentaire peut assurer le retour complet ou partiel du courant de stimulation délivré par les microélectrodes 11 sélectionnées pour la stimulation. En général, une unique surface supplémentaire 3 est prévue en commun pour toutes les microélectrodes 11. Plusieurs surfaces supplémentaires 3 peuvent être utilisées individuellement ou conjointement de préférence au même potentiel. Il peut également y avoir plusieurs supplémentaires 3, portées à des potentiels différents, par exemple d'au moins 0.1 millivolt (0.1 mV).

Le potentiel de la au moins une surface supplémentaire 3 est différent du potentiel d'au moins une microélectrode 11 de ladite pluralité déterminée de microélectrodes 11, par exemple d'au moins 0.1 millivolt (0.1 mV).

Le nombre de surfaces supplémentaires utilisées sera de préférence inférieur au dixième du nombre de microélectrodes 11 de la matrice pouvant être sélectionnées pour la stimulation dans le domaine d'application considéré.

Dans le mode de réalisation représenté à la figure 3, la surface supplémentaire est formée par une grille conductrice 3 insérée entre les microélectrodes 11 tout en entourant celles-ci. La grille 3 comporte des mailles 31 formant les zones conductrices entourant les microélectrodes 11, ces zones 31 étant reliées de manière conductrice les unes aux autres par les noeuds et branches 32 de la grille 3. La grille 3 comporte des mailles 31 disposées dans l'espace situé entre des microélectrodes voisines 11.

Dans le mode de réalisation des figures 3, 4, 5, 6 et 7, chaque maille 31 ou ouverture 36 entoure une seule microélectrode 11 associée. Chaque maille peut toutefois entourer une pluralité de microélectrodes.

Chaque microélectrode 11 de stimulation et son conducteur 21 associé sont isolés électriquement de la surface 3.

Dans les modes de réalisation des figures 3, 4, 5, 6, et 8, la surface 3 ne nécessite qu'un seul conducteur extérieur 35 pour le retour du signal de stimulation envoyé par l'une des microélectrodes 11 de stimulation au tissu vivant et une seule borne d'accès 33 pour relier la surface 3 au conducteur 35 extérieur de retour. Par conséquent, on se dispense de devoir insérer un conducteur d'accès supplémentaire entre les microélectrodes 11 de stimulation pour le retour du signal de stimulation. La matrice de microélectrodes 11 de stimulation munie de la surface 3 et de leurs accès 13 et 33 peut être prévue dans un module autonome amovible 1, ayant dans son circuit 16 d'interface électrique d'entrée-sortie avec l'extérieur les accès électriques 13 aux microélectrodes 11 et la borne d'accès 33 à la surface 3. Ce module autonome 1 peut être monté sur un réceptacle 4 comportant des bornes 41 de connexion électrique respectivement aux accès 13 des microélectrodes 11 de stimulation et la borne 35 de connexion électrique à l'accès 33. Ce module 1 peut également contenir lui-même tout ou partie de l'électronique d'enregistrement et de stimulation, notamment dans le cas d'implants.

On obtient ainsi une stimulation à la fois focale et homogène spatialement du tissu vivant autour de la microélectrode 11 stimulante. Le problème de la focalisation des stimulations est résolu de manière technologiquement très simple avec une seule contre-électrode formée par la surface 3 assurant le retour du courant, même si plusieurs microélectrodes 11 stimulent simultanément. L'invention permet ainsi d'introduire la notion de pixel de stimulation restreint à chaque microélectrode 11 de stimulation, ce qui pourra être d'une importance capitale dans la construction d'implants rétiniens, où chaque zone de la rétine doit être stimulée localement de manière non corrélée aux autres régions. De plus, l'invention permet de s'affranchir des stimulations multipolaires qui nécessitent de multiplier le nombre d'électrodes stimulantes, qui nécessitent davantage de courant pour activer les cellules, et qui créent des zones de stimulations non homogènes.

Les microélectrodes 11 ont par exemple une disposition régulière, comme par exemple en lignes et en colonnes orthogonales à la figure 3, la grille 3 pouvant alors être formée de lignes rectilignes orthogonales passant dans les espaces entre les microélectrodes 11 de stimulation. Bien entendu, les microélectrodes 11 et la grille 3 pourraient avoir toute autre disposition.

Les microélectrodes 11 de stimulation sont réparties dans une région 14 d'application du dispositif de stimulation contre le tissu vivant, cette région d'application 14 étant délimitée par les microélectrodes 15 de stimulation situées au bord de la matrice 1. L'accès 33 à la surface 3 se trouve en général à l'extérieur de la région 14 d'application des microélectrodes 11, sans passer entre les microélectrodes 11 de stimulation, contrairement aux dispositifs décrits ci-dessus de l'état de la technique. Cependant un accès direct à la surface 3 est envisageable hors plan des microélectrodes 11, par exemple à travers l'épaisseur du support des microélectrodes.

Bien entendu, une masse distante des microélectrodes 11 de stimulation et de la surface 3 peut être prévue en plus de la surface 3 pour le retour du courant de stimulation. Cependant, si la conductivité de la masse distante est plus élevée que celle de la surface 3, la stimulation pourra être moins focale qu'en absence de la masse distante.

Les sections 210 21 d'application des microélectrodes 11 de stimulation ainsi que la surface 3 peuvent être inscrites dans toute forme, par exemple plane, mais également courbe. La surface 3 peut éventuellement être insérée sur un support différent de celui des microélectrodes 11, par exemple dans le cas d'applications 3D in vivo.

Dans le mode de réalisation de la figure 4, la surface 3 est en forme de grille selon la figure 3, avec des sections 210 d'application de microélectrodes 11 de stimulation alignées suivant une ligne horizontale, chaque section 210 étant entourée par une maille 31, la grille 3 pouvant comporter d'autres mailles 32 n'entourant aucune microélectrode.

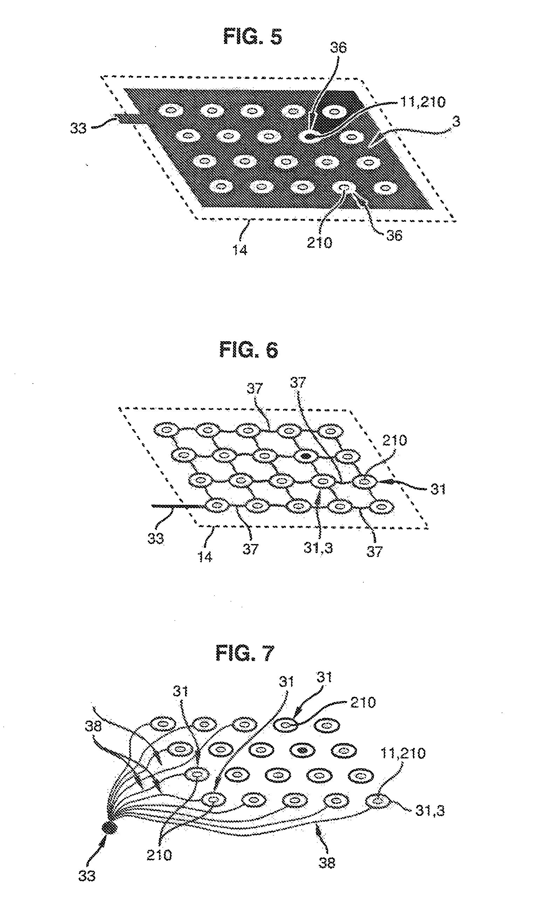

Dans le mode de réalisation représenté à la figure 5, la surface 3 supplémentaire conductrice est continue dans la région 14 avec des ouvertures 36 de passage des sections 210 des microélectrodes 11, séparées d'une distance déterminée de celle-ci et par exemple de forme correspondant à celles-ci.

Dans les modes de réalisation représentés aux figures 6 et 7, les zones conductrices 31 au voisinage des sections 210 entourent chacune une section 210 à distance de celle-ci. Les zones conductrices 31 sont par exemple de forme correspondant à celle des sections 210, par exemple annulaire pour des sections 210 circulaires.

Dans le mode de réalisation représenté à la figure 6, les zones 31 au voisinage des sections 210 sont reliées les unes aux autres par des pattes également conductrices 37, par exemple suivant deux directions sécantes dans la région 14, ces pattes 37 faisant partie de la surface 3 pour être appliquées ou non contre le tissu vivant et pouvant être rectilignes ou non.

Dans le mode de réalisation représenté à la figure 7, les zones 31 au voisinage des sections 210 sont reliées les unes aux autres par des conducteurs extérieurs 38 à la surface 3 et non appliqués contre le tissu vivant lorsque la matrice 1 et la surface 3 sont appliquées. Les conducteurs extérieurs 38 sont reliés à l'accès 33.

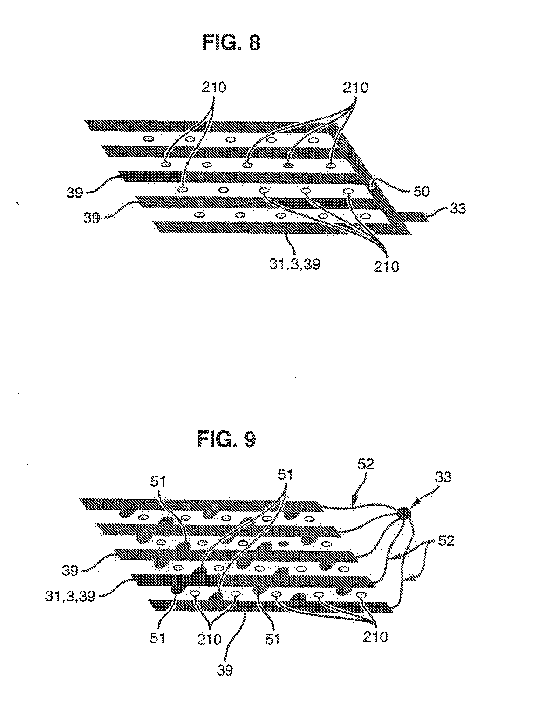

Dans le mode de réalisation représenté aux figures 8 et 9, les zones 31 au voisinage des sections 210 comportent des branches 39 s'étendant entre des sections 210 suivant un chemin continu déterminé passant à proximité de plusieurs sections 210 successives situées du même côté, par exemple entre plusieurs rangées de sections 210. Les branches 39 sont électriquement en parallèle en étant reliées les unes aux autres, par exemple par une traverse 50, cette traverse 50 faisant partie de la surface 3 pour être appliquée contre le tissu vivant et pouvant être rectilignes ou non et/ou des moyens de liaison 52 similaires aux conducteurs 38 peuvent être prévus. Les branches 39 sont par exemple rectilignes et physiquement parallèles entre elles.

Dans le mode de réalisation représenté à la figure 9, au moins une des branches 39 comporte de plus des extensions transversales 51 entre deux sections 210 successives situées du même côté de la branche 39. Ces extensions 51 sont par exemple alternes le long de la branche 39. Ces extensions. Dans l'exemple représenté, lorsque plusieurs branches 39 sont prévues, les extensions 51 d'une branche 39 ne touchent pas les autres branches 39 et en plus alternent d'une branche 39 à la branche voisine.

Bien entendu, ce qui est indiqué ci-dessus pour la surface 3 peut être prévu pour tout (ainsi que représenté) ou pour un sous-ensemble de microélectrodes 11 de la matrice 1.

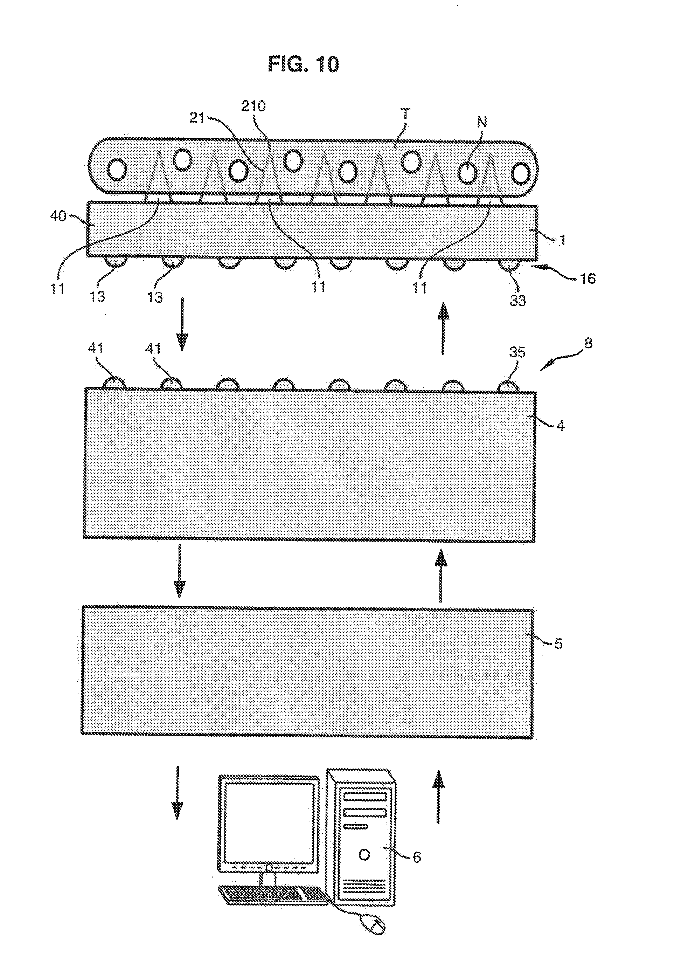

A la figure 10, le dispositif 8 de stimulation comporte un système 4 de génération de stimuli, par exemple formé par un circuit intégré à application spécifique (ASIC), relié d'une part aux bornes 13 d'accès aux microélectrodes 11 de la matrice 1 et à la borne 33 d'accès à la surface 3 et d'autre part à un système 5 d'acquisition, de stimulation et de contrôle muni d'une interface homme - machine 6, par exemple formée par un ordinateur. La matrice 1 est appliquée par sa région 14, laquelle comprend les sections 210 des microélectrodes 11 de stimulation et la surface 3, contre le tissu vivant T ayant des neurones N in vitro. On envoie à la matrice 1, par l'intermédiaire du système 4 sous la commande du système 5, des signaux de stimulation, ayant été prédéterminés sur l'interface 6, ainsi que représenté par les flèches verticales dirigées vers le haut à la figure 7. La matrice 1 peut également prélever l'activité du tissu vivant T en réponse ou non aux signaux de stimulation, qui est transmise au système 4, puis au système 5 pour pouvoir être restituée sur l'interface 6, ainsi que représenté par les flèches verticales dirigées vers le bas à la figure 7. Bien entendu, la matrice 1 de microélectrodes suivant l'invention pourrait également servir d'électrode de référence pour mesurer les variations de potentiels dans un tissu vivant reflétant les activités électriques de cellules excitables composant ce tissu. La matrice peut également être appliquée sur un tissu in vivo, pour la réhabilitation fonctionnelle notamment (audition, vision, maladies neurodégénératives ou cardiaques, par exemple) ou pour l'exploration du système nerveux central ou d'autre types de systèmes (recherche fondamentale).

La focalisation d'une stimulation extracellulaire est directement liée à la raideur du champ de potentiel autour de l'électrode de stimulation : plus ce champ est raide (i.e., croît vite avec la distance), plus il faut de courant pour stimuler à une grande distance.

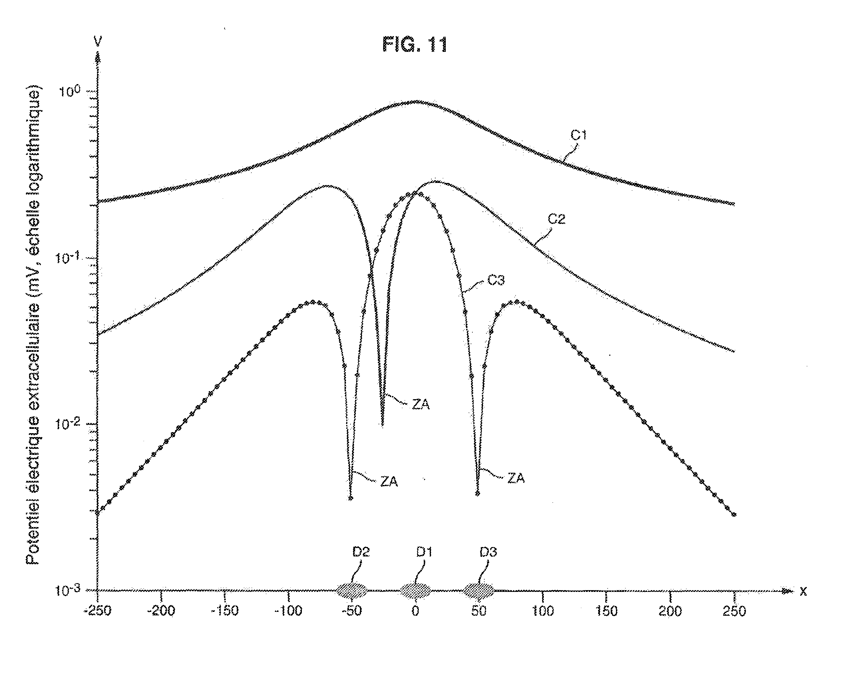

Ainsi, focaliser une stimulation revient à focaliser le champ de potentiel autour de l'électrode de stimulation. Les figures 11, 12, 13 montrent l'évolution du champ de potentiel pour différentes configurations d'électrodes.

La figure 11 montre l'évolution du potentiel V (en valeur absolue) sur une ligne passant 50 microns au-dessus des électrodes, pour des stimulations monopolaire (courbe C1), bipolaire (courbe C2), et tripolaire (courbe C3). Une stimulation monopolaire consiste à faire passer le courant entre une électrode et une masse distante, une stimulation bipolaire consiste à faire passer le courant entre deux électrodes voisines, et une stimulation tripolaire consiste à injecter un courant dans une électrode et faire revenir la moitié du courant dans deux électrodes situées de part et d'autre. Dans la figure 11, les trois disques gris D1, D2, D3 indiquent les positions à 0 µm, -50 µm et + 50 µm des électrodes alignées suivant l'axe x des abscisses dans le cas tripolaire. Dans le cas monopolaire, seule l'électrode centrale D1 est utilisée. Dans le cas bipolaire les deux électrodes de gauche D1 et D2 sont utilisées. En monopolaire, l'évolution C1 du potentiel est peu raide et donc peu focale. En bipolaire, la courbe C2 est plus raide, mais présente une zone proche des électrodes où le potentiel est très bas (zone aveugle ZA). De même en tripolaire, la courbe C3 est encore plus raide mais cette fois, on observe deux zones aveugles ZA proches des électrodes. Ainsi, les configurations multipolaires sont plus focales mais le champ n'est pas homogène autour des électrodes et présente notamment des zones aveugles où les cellules, pourtant proches des électrodes, ne seront pas stimulées.

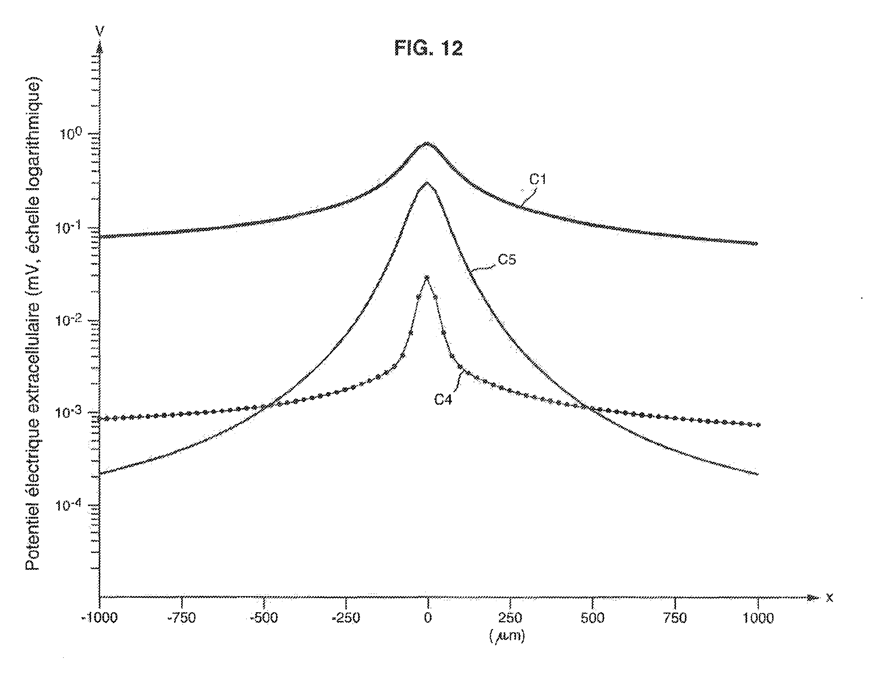

Dans un mode de réalisation de l'invention, on utilise une grille (ou un plan = grille remplie, plus généralement surface) qui passe autour de toutes les électrodes de la matrice et qui assure le retour de tout ou partie du courant de stimulation quelle que soit l'électrode qui stimule. Le potentiel de cette surface est de préférence maintenu au potentiel de la masse, bien que ceci ne soit pas une nécessité. La figure 12 montre l'évolution du potentiel V pour 3 types de configurations, en fonction de la distance x en abscisse par rapport à une microélectrode stimulante située à x = 0 µm:
- courbe C1 dans la première configuration monopolaire de la figure 11,
- courbe C4 dans une deuxième configuration selon le brevet US-A-5 411 540, dans laquelle il est prévu une seule électrode, formée par un disque intérieur conducteur de diamètre 10 microns, entouré par un anneau conducteur de diamètre extérieur 25 microns et de largeur radiale 3 microns centré sur le disque,
- courbe C5 dans une troisième configuration de la présente invention, avec une surface 3 comme en figure 5 dont les ouvertures 36 ont un diamètre de 25 microns et les électrodes centrales 11 sont des disques de diamètre 10 microns, centrés sur ces ouvertures.

Le potentiel obtenu par la courbe C5 est continu et monotone en fonction de la distance par rapport à la microélectrode stimulante. Dans la deuxième configuration, lorsque, l'anneau est au même potentiel qu'une masse distante, le champ de potentiel est identique à celui C1 généré en configuration monopolaire et la focalité de la stimulation n'est pas améliorée. Dans la deuxième configuration selon la courbe C4, lorsque l'anneau assure le retour complet du courant sans qu'aucun aucun courant ne revienne par une masse distante, la stimulation est plus focale qu'en monopolaire, mais pour un même courant délivré, le potentiel électrique est beaucoup plus bas et donc moins efficace. La présente invention avec une grille de masse selon la courbe C5 donne une stimulation très focale, et une amplitude du potentiel très proche de l'amplitude obtenue en monopolaire à proximité de l'électrode de stimulation. De plus, pour une distance supérieure à 200 microns, la grille de masse focalise mieux le potentiel que l'anneau de la deuxième configuration: l'atténuation de la courbe C5 est plus importante que celle de la courbe C4. Cette solution présente donc trois avantages par rapport à la deuxième configuration: 1) de ne pas dédoubler le nombre d'électrodes, 2) de nécessiter moins de courant pour une amplitude identique du potentiel proche de l'électrode, ce qui est important au regard du fait qu'il est difficile de concevoir des électrodes de faible taille qui ne se dégradent pas pour des courants élevés, et 3) une meilleure focalisation du potentiel à partir de quelques centaines de microns.

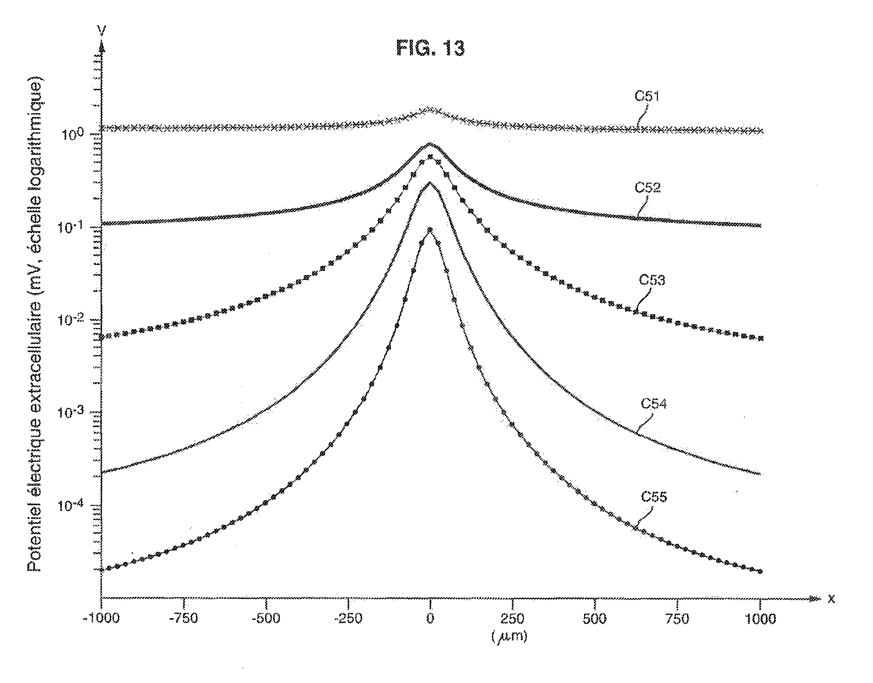

Une autre caractéristique de l'invention concerne la conductivité surfacique de l'interface entre la grille de masse ou plus généralement la surface 3 et l'électrolyte, étant précisé que l'on ne considère pas la conductivité du métal qui constitue la grille de masse, mais bien de la conductivité de l'interface électrochimique métal/liquide physiologique ou métal/tissu. Les conductivités surfaciques sont considérées pour des fréquences de signal comprises entre 10 et 100000 Hz et notamment de 100 Hz à 1000 Hz, la fréquence standard étant de 1000 Hz. La figure 13 montre l'évolution du potentiel V en fonction de la distance x en abscisse par rapport à une microélectrode stimulante située à x = 0 microns pour différentes conductivités surfaciques de la grille de masse : 40 S/m2 (courbe C51), 400 S/m2 (courbe C52), 4000 S/m2 (courbe C53), 40000 S/m2 (courbe C54), conductivité infinie (courbe C55). Plus la conductivité surfacique de la grille de masse est grande, plus la stimulation est focale. En pratique, on peut prendre des valeurs >1000 S/m2 qui sont supérieures à celles des électrodes en platine, et qui peuvent être obtenues par exemple avec d'autres matériaux ou des surfaces rugueuses, poreuses, ou fonctionnalisées (par exemple platine noir). Il est entendu que le type de conductivité considéré ici est la conductivité par unité de surface géométrique et non de surface réelle. En effet, une interface rugueuse ou poreuse aura une surface réelle (déployée/développée) plus grande qu'une même surface géométrique lisse. Des valeurs de conductivité de l'ordre de 400 S/m2 sont obtenues par exemple par des parties conductrices en platine brut blanc. Des valeurs de conductivité de l'ordre de 40000 S/m2 sont obtenues par exemple par des parties conductrices en platine traité noir.

### Liste des références citées :

[1] Microelectrode arrays for stimulation of neural slice preparations. D.A. Borkholder, J. Bao, N.I. Maluf, E.R. Perl, G.T.A. Kovacs, Journal of Neuroscience Methods 77 (1997) 61-66.
[2] Fabrication of microelectrode arrays for neural measurements from retinal tissue. W. Cunningham, K. Mathieson, F.A. Mc Ewan, A. Blue, R. McGeachy, J.A. McLeod, C. Morris-Ellis, V.O'Shea, K. M. Smith, A. Litke, M. Rahman, ournal of Physics D : Applied Physics 34 (2001) 2804-2809.
[3] Sheet conductor model of brain slices for stimulation and recording with planar electronic contacts. P. Fromherz, Eur Biophys J (2002) 31 : 228-231.
[4] Effective parameters for stimulation of dissociated cultures using multi-electrode arrays. Daniel A. Wagenaar, Jerome Pine, Steve M. Potter, Journal of Neuroscience Methods 138 (2004) 27-37.
[5] Multi-electrode stimulation and recording in the isolated retina. Andrew E. Grumet, John L. Wyatt, Joseph F. Rizzo, Journal of Neuroscience Methods 101 (2000) 31-42.
[6] A three-dimensional multi-electrode array for multi-site stimulation and recording in acute brain slices. Marc Olivier Heuschkel, Michael Fejtl, Mario Raggenbass, Daniel Bertrand, Philippe Renaud, Journal of Neuroscience Methods 114 (2002) 135-148.
[7] brevet US-A-5 411 540.
[8] demande de brevet WO 2005/087309.

## Revendications

1. Dispositif de stimulation d'un tissu vivant, comprenant une matrice (1) de microélectrodes (11) disposées selon une configuration déterminée côte à côte, chaque microélectrode comportant une section (210) d'application locale au tissu vivant et un conducteur (21) d'envoi d'un signal de stimulation et étant sélectionnable pour l'application d'un signal électrique de stimulation,
les microélectrodes (11) étant isolées l'une de l'autre,
les conducteurs (21) d'envoi de signal de stimulation étant isolés l'un de l'autre,
les sections (210) d'application locale étant isolées l'une de l'autre,
une pluralité de zones conductrices (31) étant localisées, respectivement pour une pluralité déterminée de microélectrodes (11), au voisinage de la pluralité des sections (210) d'application locale desdites microélectrodes (11) déterminées de la matrice en étant isolées de ces sections (210) d'application locale,
**caractérisé en ce que**
ladite pluralité des zones conductrices (31) font partie d'une surface conductrice (3) supplémentaire aux microélectrodes (11) et distincte des microélectrodes (11), la surface conductrice (3) supplémentaire servant à l'application en tout ou partie contre le tissu vivant,
des moyens (32, 37, 38, 50, 52) de liaison étant prévus pour assurer une liaison électrique entre ladite pluralité des zones (31) conductrices de la surface supplémentaire (3) pour toute ladite pluralité déterminée des microélectrodes (11) isolées l'une de l'autre, pour que les zones (31) conductrices de la surface supplémentaire (3) soient sensiblement équipotentielles,
la surface conductrice supplémentaire (3) étant reliée en outre à au moins un accès (33) destiné à être relié à un conducteur extérieur (35) de retour au moins partiel du signal de stimulation et étant formée pour assurer une stimulation focale à partir d'au moins une de la pluralité déterminée de microélectrodes (11) et pour servir de moyen de focalisation en commun pour plusieurs sections (210) d'application de microélectrodes (11) différentes déterminées.

2. Dispositif suivant la revendication 1, **caractérisé en ce qu'**il comporte une seule surface conductrice (3) supplémentaire.

3. Dispositif suivant l'une quelconque des revendications précédentes, **caractérisé en ce que** le nombre de surfaces supplémentaires (3) est inférieur au dixième du nombre de microélectrodes (11) de la matrice.

4. Dispositif suivant l'une quelconque des revendications 1 et 3, **caractérisé en ce qu'**il y a plusieurs surfaces supplémentaires (3), sensiblement équipotentielles.

5. Dispositif suivant l'une quelconque des revendications 1 et 3, **caractérisé en ce qu'**il y a plusieurs surfaces supplémentaires (3) à des potentiels sensiblement différents.

6. Dispositif suivant l'une quelconque des revendications précédentes, **caractérisé en ce que** le potentiel de la au moins une surface supplémentaire (3) est différent du potentiel d'au moins une microélectrode (11) de ladite pluralité déterminée de microélectrodes (11).

7. Dispositif suivant l'une quelconque des revendications précédentes, **caractérisé en ce que** la surface conductrice (3) supplémentaire est intégrée au même support que celui des microélectrodes (11).

8. Dispositif suivant l'une quelconque des revendications précédentes, **caractérisé en ce que** la surface conductrice (3) supplémentaire est intégrée sur un support différent de celui des microélectrodes (11).

9. Dispositif suivant l'une quelconque des revendications précédentes, **caractérisé en ce que** la surface conductrice (3) supplémentaire est en forme de grille, dont les zones conductrices sont formées par des mailles (31) passant autour de microélectrodes (11), les moyens de liaison étant formés sur la surface supplémentaire par les intersections (32) des mailles (31) entre elles.

10. Dispositif suivant la revendication 9, **caractérisé en ce que** lesdites mailles (31) passant autour de microélectrodes (11) entourent chacune une seule microélectrode (11).

11. Dispositif suivant l'une quelconque des revendications 9 et 10, **caractérisé en ce que** la grille (3) est formée par des lignes rectilignes sécantes.

12. Dispositif suivant l'une quelconque des revendications 9 à 11, **caractérisé en ce que** chacune des mailles (31) de la grille (3) entourant une microélectrode (11) forme un pixel de stimulation restreint à l'espace délimité par cette maille (31).

13. Dispositif suivant l'une quelconque des revendications précédentes, **caractérisé en ce que** la surface conductrice (3) supplémentaire passe entre des microélectrodes (11).

14. Dispositif suivant l'une quelconque des revendications précédentes, **caractérisé en ce que** la surface conductrice (3) supplémentaire entoure des microélectrodes (11).

15. Dispositif suivant l'une quelconque des revendications précédentes, **caractérisé en ce que** la surface conductrice (3) supplémentaire est continue avec des ouvertures (36) de passage des sections (210) d'application locale de microélectrodes (11).

16. Dispositif suivant l'une quelconque des revendications précédentes, **caractérisé en ce que** la surface conductrice (3) supplémentaire comporte des branches (39) électriquement en parallèle entre des microélectrodes (11) et passant chacune au voisinage de plusieurs microélectrodes (11).

17. Dispositif suivant l'une quelconque des revendications 1 à 16, **caractérisé en ce que** les moyens (32, 37, 50) de liaison électrique entre les différentes zones conductrices (31) sont situés au moins en partie dans la surface conductrice (3) supplémentaire.

18. Dispositif suivant l'une quelconque des revendications 1 à 16, **caractérisé en ce que** les moyens (38, 52) de liaison électrique sont situés au moins en partie à l'extérieur de la surface conductrice (3) supplémentaire, à l'intérieur ou à l'extérieur d'un support de la surface conductrice (3) supplémentaire ou dans un circuit électrique extérieur supplémentaire.

19. Dispositif suivant l'une quelconque des revendications précédentes, **caractérisé en ce que** la surface conductrice (3) supplémentaire présente une conductivité électrique surfacique d'interface avec le tissu vivant supérieure ou égale à 100 S/m² à une fréquence de 100 Hz à 1000 Hz.

20. Dispositif suivant l'une quelconque des revendications précédentes, **caractérisé en ce que** la surface conductrice (3) supplémentaire présente une conductivité électrique surfacique d'interface avec le tissu vivant supérieure ou égale à 1000 S/m² à une fréquence de 100 Hz à 1000 Hz, et de préférence supérieure ou égale à 40000 S/m² à une fréquence de 100 Hz à 1000 Hz.

21. Dispositif suivant l'une quelconque des revendications précédentes, **caractérisé en ce que** la matrice de microélectrodes (11) possède un pas d'écartement entre microélectrodes, et lesdites zones conductrices (31) de la surface supplémentaire (3) passent à une distance de la pluralité des microélectrodes (11), inférieure ou égale à cinq fois le pas d'écartement maximum entre microélectrodes, et de préférence à une distance inférieure ou égale au pas d'écartement minimum entre microélectrodes.

22. Dispositif suivant l'une quelconque des revendications précédentes, **caractérisé en ce que** lesdites zones conductrices (31) de la surface supplémentaire (3) passent à une distance de la pluralité des microélectrodes, inférieure ou égale à 500 µm.

23. Dispositif suivant l'une quelconque des revendications précédentes, **caractérisé en ce qu'**il comporte une multiplicité d'accès électriques (13) aux microélectrodes (11), qui sont associés respectivement à la multiplicité de microélectrodes (11) de la matrice (1), l'accès (33) de la surface conductrice (3) supplémentaire étant unique et distinct des accès électriques (13) des microélectrodes (11).

24. Dispositif suivant l'une quelconque des revendications 1 à 22, **caractérisé en ce qu'**il comporte une multiplicité d'accès électriques (13) aux microélectrodes (11), qui sont associés respectivement à la multiplicité de microélectrodes (11) de la matrice (1), l'accès (33) de la surface conductrice (3) supplémentaire étant multiple et distinct des accès électriques (13) des microélectrodes (11).

25. Dispositif suivant l'une quelconque des revendications précédentes, **caractérisé en ce que** les microélectrodes (15) situées au bord de la matrice (1) délimitent une région (14) d'application contre le tissu vivant et l'accès (33) de la surface (3) est situé à l'extérieur de la région (14) d'application des microélectrodes (11).

26. Dispositif suivant l'une quelconque des revendications 1 à 24, **caractérisé en ce que** les microélectrodes (15) situées au bord de la matrice (1) délimitent une région (14) d'application contre le tissu vivant et l'accès (33) de la surface (3) est situé à l'intérieur de la région (14) d'application des microélectrodes (11).

27. Dispositif suivant l'une quelconque des revendications précédentes, **caractérisé en ce qu'**il comporte en outre un premier système (4) de génération de stimulus électriques, d'amplification et de multiplexage des signaux enregistrés avec les microélectrodes, relié aux microélectrodes (11) et à l'accès (33) de la surface (3), un deuxième système (5) d'acquisition et de contrôle muni d'une interface homme - machine (6) pour la commande du premier système (4) afin d'envoyer à au moins l'une présélectionnée des microélectrodes (11) un signal de stimulation prédéterminé sur l'interface homme - machine (6), et de recueillir la réponse du tissu vivant au signal de stimulation et la restituer sur l'interface homme - machine (6).

28. Utilisation in vitro du dispositif suivant l'une quelconque des revendications précédentes, pour la mise en oeuvre en contact avec l'un parmi une préparation cellulaire, un explant, un système de laboratoire, un organe vivant isolé, une partie d'organe vivant isolé.

29. Ensemble amovible destiné à être monté dans le dispositif suivant l'une quelconque des revendications 1 à 27, **caractérisé en ce qu'**il comporte sur un même module amovible ou répartis sur plusieurs modules amovibles séparés la matrice (1) de microélectrodes, ladite surface (3) supplémentaire, et un circuit (16) d'interface électrique d'entrée-sortie pour la connexion électrique des microélectrodes (11) et de la surface (3) supplémentaire avec l'extérieur, comportant une multiplicité de bornes d'accès électriques (13) à respectivement la multiplicité de microélectrodes (11) de la matrice (1) et une borne d'accès (33) de surface (3) supplémentaire, distincte des bornes d'accès électriques (13) des microélectrodes (11).

30. Utilisation du dispositif suivant l'une quelconque des revendications 1 à 27 pour l'enregistrement de signaux émis par un tissu vivant de l'un parmi une préparation cellulaire, un explant, un système de laboratoire, un organe vivant isolé, une partie d'organe vivant isolé.

## Patentansprüche

1. Vorrichtung zur Stimulation eines lebenden Gewebes, die eine Matrix (1) von Mikroelektroden (11) umfasst, die entsprechend einer festgelegten Konfiguration nebeneinander angeordnet sind, wobei jede Mikroelektrode einen Abschnitt (210) zur lokalen Anwendung auf das lebende Gewebe und eine Leitung (21) zur Zuführung eines Stimulationssignals umfasst und für die Anwendung eines elektrischen Stimulationssignals auswählbar ist, wobei
die Mikroelektroden (11) voneinander isoliert sind,
die Leitungen (21) zur Zuführung eines Stimulationssignals voneinander isoliert sind,
die Abschnitte (210) zur lokalen Anwendung voneinander isoliert sind,
eine Anzahl leitender Bereiche (31), jeweils für eine festgelegte Anzahl Mikroelektroden (11), benachbart zu der Anzahl Abschnitte (210) zur lokalen Anwendung der festgelegten Mikroelektroden (11) der Matrix positioniert sind, wobei sie von den Abschnitten (210) zur lokalen Anwendung isoliert sind,
**dadurch gekennzeichnet, dass**
die Anzahl leitender Bereiche (31) Teil einer leitenden Fläche (3) sind, die zusätzlich zu den Mikroelektroden (11) und von den Mikroelektroden (11) verschieden ist, wobei die zusätzliche leitende Fläche (3) dazu dient, ganz oder teilweise auf das lebende Gewebe aufgesetzt zu werden,
Verbindungsmittel (32, 37, 38, 50, 52) vorgesehen sind, um eine elektrische Verbindung zwischen der Anzahl leitender Bereiche (31) der zusätzlichen Fläche (3) bereitzustellen, und dies für die gesamte festgelegte Anzahl voneinander isolierter Mikroelektroden (11), damit die leitenden Bereiche (31) der zusätzlichen Fläche (3) im Wesentlichen equipotential sind,
die zusätzliche leitende Fläche (3) außerdem mit wenigstens einem Anschluss (33) verbunden ist, der dafür vorgesehen ist, an eine externe Leitung (35) zur wenigstens teilweisen Rückführung des Stimulationssignals angeschlossen zu werden, und dafür ausgebildet ist, ausgehend von wenigstens einer der festgelegten Anzahl Mikroelektroden (11) eine fokussierte Stimulation bereitzustellen und als gemeinsames Fokussierungsmittel für mehrere Anwendungsabschnitte (210) von verschiedenen und festgelegten Mikroelektroden (11) zu dienen.

2. Vorrichtung nach Anspruch 1, **dadurch gekennzeichnet, dass** sie eine einzige zusätzliche leitende Fläche (3) umfasst.

3. Vorrichtung nach einem der vorhergehenden Ansprüche, **dadurch gekennzeichnet, dass** die Zahl zusätzlicher Flächen (3) kleiner ist als ein Zehntel der Zahl Mikroelektroden (11) der Matrix.

4. Vorrichtung nach einem der Ansprüche 1 bis 3, **dadurch gekennzeichnet, dass** mehrere zusätzliche Flächen (3) vorhanden sind, die im Wesentlichen equipotential sind.

5. Vorrichtung nach einem der Ansprüche 1 bis 3, **dadurch gekennzeichnet, dass** mehrere zusätzliche Flächen (3) mit im Wesentlichen verschiedenen Potentialen vorhanden sind.

6. Vorrichtung nach einem der vorhergehenden Ansprüche, **dadurch gekennzeichnet, dass** das Potential der wenigstens einen zusätzlichen Fläche (3) vom Potential wenigstens einer Mikroelektrode (11) der festgelegten Anzahl Mikroelektroden (11) verschieden ist.

7. Vorrichtung nach einem der vorhergehenden Ansprüche, **dadurch gekennzeichnet, dass** die zusätzliche leitende Fläche (3) in denselben Träger integriert ist wie die Mikroelektroden (11).

8. Vorrichtung nach einem der vorhergehenden Ansprüche, **dadurch gekennzeichnet, dass** die zusätzliche leitende Fläche (3) in einen Träger integriert ist, der verschieden von demjenigen der Mikroelektroden (11) ist.

9. Vorrichtung nach einem der vorhergehenden Ansprüche, **dadurch gekennzeichnet, dass** die zusätzliche leitende Fläche (3) die Form eines Gitters hat, dessen leitende Bereiche durch Maschen (31) gebildet werden, die um Mikroelektroden (11) herum verlaufen, wobei die Verbindungsmittel auf der zusätzlichen Fläche durch die Kreuzungen (32) der Maschen (31) miteinander gebildet werden.

10. Vorrichtung nach Anspruch 9, **dadurch gekennzeichnet, dass** von den Maschen (31), die um Mikroelektroden (11) herum verlaufen, jede um eine einzige Mikroelektrode (11) herum verläuft.

11. Vorrichtung nach einem der Ansprüche 9 und 10, **dadurch gekennzeichnet, dass** das Gitter (3) durch schneidende geradlinige Linien gebildet wird.

12. Vorrichtung nach einem der Ansprüche 9 bis 11, **dadurch gekennzeichnet, dass** jede der Maschen (31) des Gitters (3), die um eine Mikroelektrode (11) herum verläuft, ein Stimulationspixel bildet, das auf den von dieser Masche (31) begrenzten Raum eingeschränkt ist.

13. Vorrichtung nach einem der vorhergehenden Ansprüche, **dadurch gekennzeichnet, dass** die zusätzliche leitende Fläche (3) zwischen Mikroelektroden (11) hindurch verläuft.

14. Vorrichtung nach einem der vorhergehenden Ansprüche, **dadurch gekennzeichnet, dass** die zusätzliche leitende Fläche (3) Mikroelektroden (11) umgibt.

15. Vorrichtung nach einem der vorhergehenden Ansprüche, **dadurch gekennzeichnet, dass** die zusätzliche leitende Fläche (3) mit Öffnungen (36) für den Durchgang der Abschnitte (210) zur lokalen Anwendung der Mikroelektroden (11) versehen ist.

16. Vorrichtung nach einem der vorhergehenden Ansprüche, **dadurch gekennzeichnet, dass** die zusätzliche leitende Fläche (3) Äste (39) umfasst, die zwischen Mikroelektroden (11) elektrisch parallel geschaltet sind und von denen jeder benachbart zu mehreren Mikroelektroden (11) verläuft.

17. Vorrichtung nach einem der Ansprüche 1 bis 16, **dadurch gekennzeichnet, dass** die Mittel (32, 37, 50) zur elektrischen Verbindung zwischen den verschiedenen leitenden Bereichen (31) wenigstens teilweise in der zusätzlichen leitenden Fläche (3) angeordnet sind.

18. Vorrichtung nach einem der Ansprüche 1 bis 16, **dadurch gekennzeichnet, dass** die Mittel (38, 52) zur elektrischen Verbindung wenigstens teilweise außerhalb der zusätzlichen leitenden Fläche (3), innerhalb oder außerhalb eines Trägers der zusätzlichen leitenden Fläche (3) oder in einer zusätzlichen äußeren elektrischen Schaltung angeordnet sind.

19. Vorrichtung nach einem der vorhergehenden Ansprüche, **dadurch gekennzeichnet, dass** die zusätzliche leitende Fläche (3) eine elektrische Oberflächenleitfähigkeit an der Schnittstelle zum lebenden Gewebe hat, die größer oder gleich 100 S/m² bei einer Frequenz von 100 Hz bis 1000 Hz ist.

20. Vorrichtung nach einem der vorhergehenden Ansprüche, **dadurch gekennzeichnet, dass** die zusätzliche leitende Fläche (3) eine elektrische Oberflächenleitfähigkeit an der Schnittstelle zum lebenden Gewebe hat, die größer oder gleich 1000 S/m² bei einer Frequenz von 100 Hz bis 1000 Hz ist und vorzugsweise größer oder gleich 40000 S/m² bei einer Frequenz von 100 Hz bis 1000 Hz.

21. Vorrichtung nach einem der vorhergehenden Ansprüche, **dadurch gekennzeichnet, dass** die Mikroelektrodenmatrix (11) ein Abstandsmaß zwischen Mikroelektroden umfasst, wobei die leitenden Bereiche (31) der zusätzlichen Fläche (3) in einem Abstand von der Anzahl Mikroelektroden (11) verlaufen, der kleiner oder gleich dem Fünffachen des maximalen Abstandsmaßes zwischen Mikroelektroden ist und vorzugsweise in einem Abstand, der kleiner oder gleich dem minimalen Abstandsmaß zwischen Mikroelektroden ist.

22. Vorrichtung nach einem der vorhergehenden Ansprüche, **dadurch gekennzeichnet, dass** die leitenden Bereiche (31) der zusätzlichen Fläche (3) in einem Abstand von der Anzahl Mikroelektroden verlaufen, der kleiner oder gleich 500 µm ist.

23. Vorrichtung nach einem der vorhergehenden Ansprüche, **dadurch gekennzeichnet, dass** sie eine Vielzahl elektrischer Anschlüsse (13) für die Mikroelektroden (11) umfasst, die jeweils der Vielzahl Mikroelektroden (11) der Matrix (1) zugeordnet sind, wobei der Anschluss (33) der zusätzlichen leitenden Fläche (3) nur einmal vorhanden und von den elektrischen Anschlüssen (13) der Mikroelektroden (11) verschieden ist.

24. Vorrichtung nach einem der Ansprüche 1 bis 22, **dadurch gekennzeichnet, dass** sie eine Vielzahl elektrischer Anschlüsse (13) für die Mikroelektroden (11) umfasst, die jeweils der Vielzahl Mikroelektroden (11) der Matrix (1) zugeordnet sind, wobei der Anschluss (33) der zusätzlichen leitenden Fläche (3) mehrfach vorhanden und von den elektrischen Anschlüssen (13) der Mikroelektroden (11) verschieden ist.

25. Vorrichtung nach einem der vorhergehenden Ansprüche, **dadurch gekennzeichnet, dass** die Mikroelektroden (15), die sich am Rand der Matrix (1) befinden, einen Bereich (14) zum Aufsetzen auf das lebende Gewebe abgrenzen, wobei der Anschluss (33) der Fläche (3) sich außerhalb des Bereichs (14) zum Aufsetzen der Mikroelektroden (11) befindet.

26. Vorrichtung nach einem der Ansprüche 1 bis 24, **dadurch gekennzeichnet, dass** die Mikroelektroden (15), die sich am Rand der Matrix (1) befinden, einen Bereich (14) zum Aufsetzen auf das lebende Gewebe abgrenzen, wobei der Anschluss (33) der Fläche (3) sich innerhalb des Bereichs (14) zum Aufsetzen der Mikroelektroden (11) befindet.

27. Vorrichtung nach einem der vorhergehenden Ansprüche, **dadurch gekennzeichnet, dass** es außerdem Folgendes umfasst: ein erstes System (4) zum Erzeugen eines elektrischen Stimulus, zur Verstärkung und zum Multiplexen der mit den Mikroelektroden aufgenommenen Signale, das mit den Mikroelektroden (11) und dem Anschluss (33) der Fläche (3) verbunden ist, und ein zweites System (5) zum Erfassen und zur Steuerung, das mit einer Mensch-Maschine-Schnittstelle (6) zur Steuerung des ersten Systems (4) ausgestattet ist, um an wenigstens eine vorausgewählte der Mikroelektroden (11) ein auf der Mensch-Maschine-Schnittstelle (6) vorgegebenes Stimulationssignal zu senden und die Antwort des lebenden Gewebes auf das Stimulationssignal aufzunehmen und sie auf der Mensch-Maschine-Schnittstelle (6) wiederzugeben.

28. In-Vitro-Verwendung der Vorrichtung nach einem der vorhergehenden Ansprüche, um sie in Kontakt mit einem unter den folgenden Objekten einzusetzen: ein Zellpräparat, ein Explant, ein Laborsystem, ein isoliertes lebendes Organ, ein Teil eines isolierten lebenden Organs.

29. Abnehmbare Einheit, die dafür vorgesehen ist, in der Vorrichtung nach einem der Ansprüche 1 bis 27 montiert zu werden, **dadurch gekennzeichnet, dass** sie auf einem einzigen abnehmbaren Modul oder verteilt auf mehrere getrennte abnehmbare Module Folgendes umfasst: die Mikroelektrodenmatrix (1), die zusätzliche Fläche (3) und eine elektrische Eingangs-Ausgangs-Schnittstellenschaltung (16) für die elektrische Verbindung der Mikroelektroden (11) und der zusätzlichen Fläche (3) mit dem Äußeren, wobei diese Folgendes umfasst: eine Vielzahl elektrischer Anschlusspunkte (13) für jeweils die Vielzahl Mikroelektroden (11) der Matrix (1) und einen Anschlusspunkt (33) für die zusätzliche Fläche (3), der von den elektrischen Anschlusspunkten (13) für die Mikroelektroden (11) verschieden ist.

30. Verwendung der Vorrichtung nach einem der Ansprüche 1 bis 27 für das Aufzeichnen von Signalen, die von einem lebenden Gewebe ausgegeben werden, bei dem es sich um eines der folgenden handelt: ein Zellpräparat, ein Explant, ein Laborsystem, ein isoliertes lebendes Organ, ein Teil eines isolierten lebenden Organs.

## Claims

1. Device to stimulate a living tissue, comprising an array (1) of microelectrodes (11) arranged in a determined configuration side-by-side, each microelectrode comprising a section (210) for local application to the living tissue and a conductor (21) for sending a stimulation signal, wherein each microelectrode can be selected for application of an electric stimulation signal,
the microelectrodes (11) being insulated from each other,
the conductors (21) for sending a stimulation signal being insulated from each other,
the local application sections (210) being insulated from each other,
a plurality of conductive zones (31) being located, respectively for a determined plurality of microelectrodes (11), in the vicinity of the plurality of local application sections (210) of said determined microelectrodes (11) of the array and being insulated from these local application sections (210),
**characterized in that**:
said plurality of conductive zones (31) belongs to a conductive surface (3) supplemental to the microelectrodes (11) and separate from the microelectrodes (11), the supplemental conductive surface (3) being used for all or part of the application against the living tissue,
connection means (32, 37, 38, 50, 52) being provided to ensure electric connection between said plurality of conductive zones (31) of the supplemental surface (3) for all said determined plurality of microelectrodes (11) insulated from one another, so that the conductive zones (31) of the supplemental surface (3) are substantially equipotential,
the supplemental conductive surface (3) also being connected to at least one port (33) intended to be connected to an external return conductor (35) returning at least part of the stimulation signal and being formed to ensure focal stimulation from at least one of the determined plurality of microelectrodes (11) and to act as common focusing means for several application sections (210) of different determined microelectrodes (11).

2. Device according to claim 1, **characterized in that** it comprises a single supplemental conductive surface (3).

3. Device according to any of the preceding claims, **characterized in that** the number of supplemental surfaces (3) is less than one tenth of the number of microelectrodes (11) in the array.

4. Device according to any of claims 1 to 3, **characterized in that** there are several supplemental surfaces (3) that are substantially equipotential.

5. Device according to either of claims 1 and 3, **characterized in that** there are several supplemental surfaces (3) at substantially different potentials.

6. Device according to any of the preceding claims, **characterized in that** the potential of the at least one supplemental surface (3) is different from the potential of at least one microelectrode (11) of said determined plurality of microelectrodes (11).

7. Device according to any of the preceding claims, **characterized in that** the supplemental conductive surface (3) is integrated in the same support as that of the microelectrodes (11).

8. Device according to any of the preceding claims, **characterized in that** the supplemental conductive surface (3) is integrated on a different support to the support of the microelectrodes (11).

9. Device according to any of the preceding claims, **characterized in that** the supplemental conductive surface (3) is in the form of a grid whose conductive zones are formed by meshes (31) passing around microelectrodes (11), the connection means being formed on the supplemental surface by the intersections (32) of the meshes (31) with each other.

10. Device according to claim 9, **characterized in that** said meshes (31) passing around microelectrodes (11) each surround a single microelectrode (11).

11. Device according to either of claims 9 and 10, **characterized in that** the grid (3) is formed by rectilinear secant lines.

12. Device according to any of claims 9 to 11, **characterized in that** each of the meshes (31) of the grid (3) surrounding a microelectrode (11) form a stimulation pixel restricted to the space delimited by this mesh (31).

13. Device according to any of the preceding claims, **characterized in that** the supplemental conductive surface (3) passes between microelectrodes (11).

14. Device according to any of the preceding claims, **characterized in that** the supplemental conductive surface (3) surrounds microelectrodes (11).

15. Device according to any of the preceding claims, **characterized in that** the supplemental conductive surface (3) is continuous with openings (36) for passing of the local application sections (210) of microelectrodes (11).

16. Device according to any of the preceding claims, **characterized in that** the supplemental conductive surface (3) comprises branches (39) electrically parallel between microelectrodes (11) and each passing in the vicinity of several microelectrodes (11).

17. Device according to any of claims 1 to 16, **characterized in that** the electric connection means (32, 37, 50) between the different conductive zones (31) are located at least partly in the supplemental conductive surface (3).

18. Device according to any of claims 1 to 16, **characterized in that** the electric connection means (38, 52) are located at least partly outside the supplemental conductive surface (3), inside or outside a support of the supplemental conductive surface (3) or in an supplemental external electric circuit.

19. Device according to any of the preceding claims, **characterized in that** the supplemental conductive surface (3) has an interface surface electric conductivity with the living tissue of 100 S/m² or higher, at a frequency of 100 Hz to 1,000 Hz.

20. Device according to any of the preceding claims, **characterized in that** the supplemental conductive surface (3) has an interface surface electric conductivity with the living tissue of 1,000 S/m² or higher at a frequency of 100 Hz to 1,000 Hz, and preferably equal to or higher than 40,000 S/m² at a frequency of 100 Hz to 1,000 Hz.

21. Device according to any of the preceding claims, **characterized in that** the array of microelectrodes (11) has a pitch distance between microelectrodes, and said conductive zones (31) of the supplemental surface (3) pass at a distance from the plurality of microelectrodes (11) that is equal to or less than five times the maximum pitch distance between microelectrodes, and preferably at a distance equal to or less than the minimum pitch distance between microelectrodes.

22. Device according to any of the preceding claims, **characterized in that** said conductive zones (31) of the supplemental surface (3) pass at a distance from the plurality of microelectrodes equal to or less than 500 µm.

23. Device according to any of the preceding claims, **characterized in that** it comprises a multiplicity of electric ports (13) for access to the microelectrodes (11) which are respectively associated with the multiplicity of microelectrodes (11) of the array (1), the port (33) of the supplemental conductive surface being single and separate from the electric ports (13) of the microelectrodes (11).

24. Device according to any of claims 1 to 22, **characterized in that** it comprises a multiplicity of electric ports (13) for access to the microelectrodes (11) which are respectively associated with the multiplicity of microelectrodes (11) of the array (1), the port (33) of the supplemental conductive surface (3) being multiple and separate from the electric ports (13) of the microelectrodes (11).

25. Device according to any of the preceding claims, **characterized in that** the microelectrodes (15) located on the edge of the array (1) delimit a region (14) for application against the living tissue, and the port (33) of surface (3) is positioned outside the application region (14) of the microelectrodes (11).

26. Device according to any of claims 1 to 24, **characterized in that** the microelectrodes (15) located on the edge of the array (1) delimit a region (14) for application against the living tissue, and the port (33) of the surface (3) is positioned inside the application region (14) of the microelectrodes (11).

27. Device according to any of the preceding claims, **characterized in that** it further comprises a first system (4) for the generation of electric stimuli and the amplification and multiplexing of signals recorded with the microelectrodes, connected to the microelectrodes (11) and to the port (33) of the surface (3), a second system (5) for acquisition and control provided with a man-machine interface (6) to control the first system (4) so as to send to at least one preselected microelectrode (11) a stimulation signal pre-determined on the man-machine interface (6) and so as to collect the response of the living tissue to the stimulation signal and to reproduce it on the man-machine interface (6).

28. *In vitro* use of the device according to any of the preceding claims for its contacting with a cell preparation, an explant, a laboratory system, an isolated living organ, a part of an isolated living organ.

29. Removable assembly intended to be mounted in the device according to any of claims 1 to 27 **characterized in that**, on one same removable module or distributed over several separate removable modules, it comprises the array (1) of microelectrodes, said supplemental surface (3), an input-output electric interface circuit (16) for electric connection of the microelectrodes (11) and of the supplemental surface (3) with the outside, comprising a multiplicity of electric terminals (13) for access to respectively the multiplicity of microelectrodes (11) of the array (1) and a terminal (33) for access of the supplemental surface (3) separate from the electric terminals (13) of the microelectrodes (11).

30. Use of the device according to any of claims 1 to 27 for the recording of signals emitted by a living tissue, wherein the living tissue is one among a cell preparation, an explant, a laboratory system, an isolated living organ, a part of an isolated living organ.
